# EUROPEAN PATENT SPECIFICATION

(11) **EP 3 870 180 B1**
(45) Date of publication and mention of the grant of the patent: **22.11.2023**
(21) Application number: 19801226.2
(22) Date of filing: 25.10.2019
(51) Int. Cl.: A61K 31/519, A61K 31/445, A61K 31/7004, A61K 45/06, A61K 9/00, A61P 19/02

(54) **COMBINATION COMPRISING SILDENAFIL FOR USE IN THE TREATMENT OF OSTEOARTHRITIS**
KOMBINATION ENTHALTEND SILDENAFIL ZUR VERWENDUNG BEI DER BEHANDLUNG VON OSTEOARTHRITIS
COMBINAISON COMPRENANT LE SILDÉNAFIL DESTINÉE À ÊTRE UTILISÉE DANS LE TRAITEMENT DE L'OSTÉOARTHRITE

(30) Priority: 26.10.2018 SE 1851333
(43) Date of publication of application: 01.09.2021
(60) Divisional application: 23202770.6
(73) Proprietor: Artroa AB, 436 40 Askim (SE)
(72) Inventor: SKIÖLDEBRAND, Eva, 412 67 GÖTEBORG (SE); HANSSON RÖNNBÄCK, Elisabeth, 436 39 ASKIM (SE)
(74) Representative: Noréns Patentbyrå AB
(86) International application number: PCT/EP2019/079170
(87) International publication number: WO 2020/084113

(56) References cited:
- EP-A1- 0 967 214
- WO-A1-99/01114
- WO-A1-2013/122855
- WO-A2-2005/013947
- US-A1- 2004 248 891
- US-A1- 2009 131 442
- FAC ROCHA ET AL: "Tadalafil analgesia in experimental arthritis involves suppression of intra-articular TNF release", BRITISH JOURNAL OF PHARMACOLOGY, vol. 164, no. 2b, 28 August 2011 (2011-08-28), pages 828-835, XP55669213, UK ISSN: 0007-1188, DOI: 10.1111/j.1476-5381.2011.01469.x
- DEITERS BARTHOLD ET AL: "Inhibition of hyaluronan export reduces collagen degradation in interleukin-1 treated cartilage", ARTHRITIS RESEARCH AND THERAPY, BIOMED CENTRAL, LONDON, GB, vol. 10, no. 1, 18 January 2008 (2008-01-18), page R8, XP021041175, ISSN: 1478-6354
- MIGLIORE ALBERTO ET AL: "Comparative, double-blind, controlled study of intra-articular hyaluronic acid (HyalubrixÂ ) injections versus local anesthetic in osteoarthritis of the hip", ARTHRITIS RESEARCH AND THERAPY, BIOMED CENTRAL, LONDON, GB, vol. 11, no. 6, 9 December 2009 (2009-12-09), page R183, XP021070708, ISSN: 1478-6354
- FADI HASSAN ET AL: "The effectiveness of prolotherapy in treating knee osteoarthritis in adults: a systematic review", BRITISH MEDICAL BULLETIN., vol. 122, no. 1, 4 March 2017 (2017-03-04) , pages 91-108, XP55669219, GB ISSN: 0007-1420, DOI: 10.1093/bmb/ldx006
- ELISABETH HANSSON ET AL: "Anti-inflammatory effects induced by ultralow concentrations of bupivacaine in combination with ultralow concentrations of sildenafil (Viagra) and vitamin D3 on inflammatory reactive brain astrocytes", PLOS ONE, vol. 14, no. 10, 9 October 2019 (2019-10-09), page e0223648, XP055669425, DOI: 10.1371/journal.pone.0223648

## Description

### Field of the invention

This invention is related sildenafil for use in the treatment of osteoarthritis in humans, horse, dog or cat, where sildenafil is administered together with a local anaesthetic of the amino amide type and glucose.

### Background

Osteoarthritis (OA) is a low-grade chronic systemic inflammatory disease that results from breakdown of joint cartilage and underlying bone. The disease proceeds from an early phase which often is characterized by inflammation and disorganization of matrix proteins in the affected joints, to a more severe phase with damage to the underlying bone. The main symptom is pain in the joint. Osteoarthritis is a major clinical problem as it affects around 3.8% of the human population, and it is the major disease behind severe joint pain. It is estimated that 50% of NSAID pain killer prescriptions are related to OA. It should be noted that osteoarthritis is a completely different disease from rheumatoid arthritis, which is a rare autoimmune disease.

Osteoarthritis in horses is a great problem for horse owners. The principal reason for having a horse is to be able to use it, and often to compete with it. OA is the most frequent reason for not being able to use horses and accounts for the greatest single economic loss in the horse industry.

OA in horses and humans are caused by similar mechanisms. In both humans and horses OA proceeds from an early stage characterized by inflammation over months and years to a later stage with extensive tissue damage (Goldring, M.B., Otero M. Current Opinion in Rheumatology (2011) 23(5):471). OA disease mechanisms in humans and horses are also very similar on the molecular level (Stenberg J, Rüetschi U, Skiöldebrand E, Kärrholm J, Lindahl A. Proteome Sci. (2013) Oct 4;11(1):43) (Svala E, Löfgren M, Sihlbom C, Rüetschi U, Lindahl A, Ekman S, Skiöldebrand E. Connect Tissue Res. (2015);56(4):315-25). Intracellular calcium signalling is increased in inflammatory chondrocytes (Skiöldebrand E, Thorfe A, Björklund U, Johansson P, Wickelgren R, Lindahl A, Hansson E. Heliyon. (2018) Feb 1;4(1):e00525).

Treatment of OA in humans is today limited to changes in life style (such as weight loss and exercise) and the use of analgesics, and in severe cases, joint replacement surgery. Joint injection of glucocorticoids (such as hydrocortisone) leads to short term pain relief that may last between a few weeks and a few months.

OA in horses may be treated with for example injection of glucocorticoids, hyaluronic acid or autologous conditioned serum (ACS).

US 2009/131442 discloses the treatment of osteoarthritis (OA) and bone pain caused by OA with a PDE5 inhibitor, e.g. sildenafil. Administration can be intravenous, and to a non-human mammal, e.g. for veterinary applications. Combination with other drugs is possible.

WO 2013/122855 discloses the use of a PDE5 inhibitor, such as sildenafil, for treating a lymphatic transport dysfunction. The PDE5 inhibitor can be injected in the joint of the subject at a dose of 0.1-1 mg in a single or divided doses. Dextrose can be a carrier for parenteral administration.

Migliore et al "Comparative, double-blind, controlled study of intraarticular hyaluronic acid (HyalubrixA ) injections versus local anesthetic in osteoarthritis of the hip", ARTHRITIS RESEARCH AND THERAPY, BIOMED CENTRAL, LONDON, GB, vol. 11, no. 6, 9 December 2009 (2009-12-09), page R183, discloses that intra-arterial injection of a local anesthetic, such as mepivacaine, shows good efficacy in the treatment of hip OA.

WO 99/01114 describes that a sustained release intra-articular formulation of bupivacaine has therapeutic benefit in osteoarthritic joints.

Fadi et al: "The effectiveness of prolotherapy in treating knee osteoarthritis in adults: a systematic review", BRITISH MEDICAL BULLETIN., vol. 122, no. 1, 4 March 2017 (2017-03-04), pages 91-108, discloses the treatment of knee OA with dextrose (= glucose).

However, no disease-modifying drugs exists, for humans or horses. Thus, there is a need for improved treatment of OA.

This invention solves these and other problems.

### Summary of invention

The reference to methods of treatment in this description are to be interpreted as references to the compounds, pharmaceutical compositions and medicaments of the present invention for use in a method of treatment of the human or animal body by therapy.

The inventors have surprisingly found that sildenafil in combination with a amino amide type local anaesthetic and glucose, in particular at low concentrations of sildenafil, can be used in the treatment of OA. Sildenafil (CAS number 139755-83-2) is typically used for treating erectile dysfunction and is sold under the trademark Viagra^{®}. Surprisingly, low doses of sildenafil and sildenafil analogues are capable of inhibiting intracellular calcium signalling, in particular ATP-evoked intracellular calcium signalling. Without being bound by any theory it is believed that sildenafil is able to achieve a balance between intercellular calcium signalling through gap junctions and extracellular signalling mediated by extracellular ATP. The ATP-evoked calcium signalling is important due to increased ATP release and subsequent activation of purinergic receptors.

In a first aspect of the invention there is provided sildenafil for use in the treatment of osteoarthritis in humans, horse, dog or cat, where sildenafil is administered together with a local anaesthetic of the amino amide type and glucose. The dose of sildenafil may be a low dose, for example from 0.01 mg to 10 mg, or more preferred 0.01 mg to 0.1 mg. The dose is preferably administered at least once, more preferably at least twice. The dose may be administered one, two or three times. In a preferred embodiment the dose is administered once, in an even more preferred embodiment two times. In one embodiment sildenafil is be injected into the affected joint, in particular one, two or three times.

In a preferred embodiment the local anaesthetic is mepivacaine and the dose of sildenafil is from 0.01 mg to 0.1 mg, the dose of glucose is from 100 mg to 1000 mg and the dose of mepivacaine is from 10 mg to 200 mg. In one embodiment the dose of glucose is from 150 mg to 400 mg and the dose of mepivacaine is from 10 mg to 30 mg.

In a second aspect of the invention there is provided a pharmaceutical composition comprising sildenafil, glucose and mepivacaine. The pharmaceutical composition may be an injection solution.

### Figures

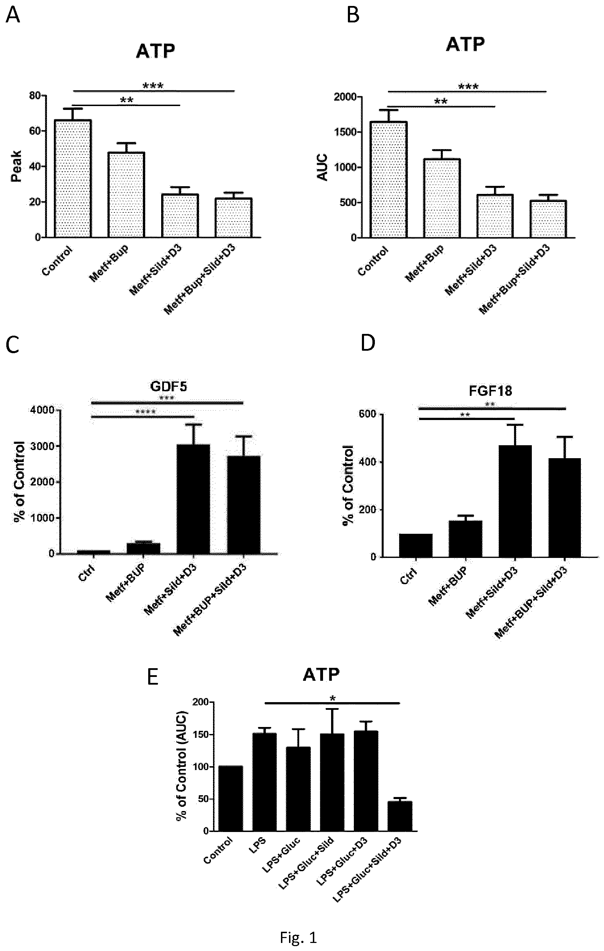

Figs. 1A-E are graphs showing effects of treatment of bupivacaine and sildenafil on chondrocytes.

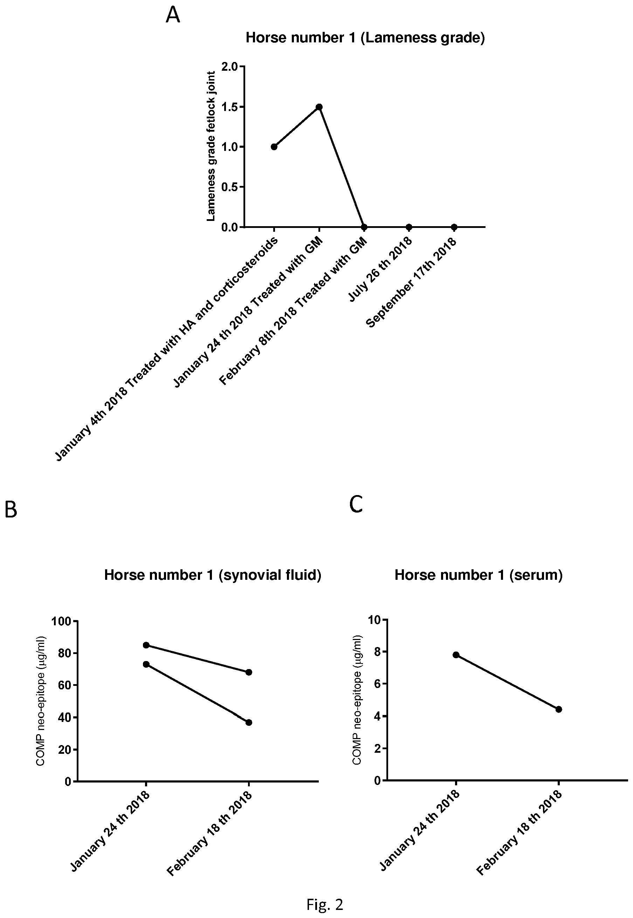

Fig. 2 shows the effect of low dose mepivacaine and glucose treatment of a horse with OA. Fig. 2A shows clinical lameness grade of carpal joints after treatment with glucose/mepivacaine. Fig. 2B is a graph that shows decreased concentration of COMP neo-epitope in synovial fluid after treatment with glucose/mepivacaine (GM). Fig. 2C is a graph that shows decreased concentration of COMP neo-epitope in serum after treatment with glucose/mepivacaine and during rehabilitation.

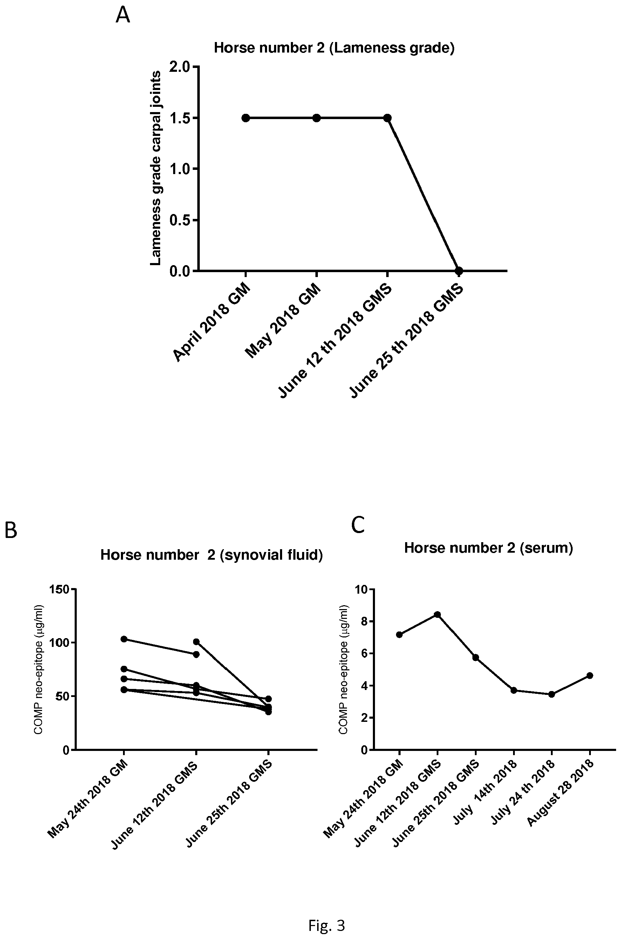

Fig. 3 shows the effect of sildenafil treatment of a horse with OA. Fig. 3A shows clinical lameness grade of carpal joints after treatment with glucose/mepivacaine/sildenafil (GMS). Fig. 3B is a graph that shows decreased concentration of COMP neo-epitope in synovial fluid after treatment with glucose/mepivacaine/sildenafil (GMS). Fig. 3C is a graph that shows decreased concentration of COMP neo-epitope in serum after treatment with glucose/ mepivacaine /sildenafil (GMS) and during rehabilitation.

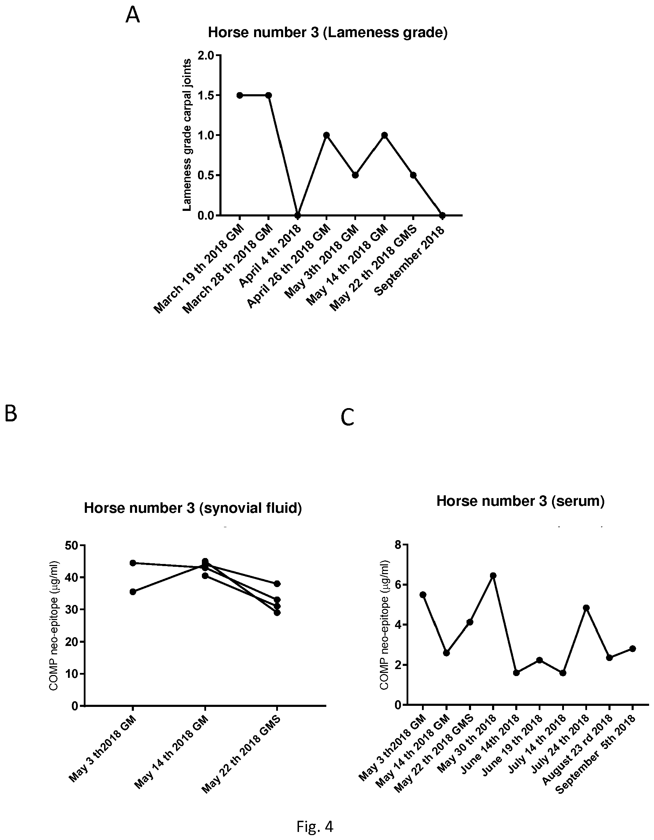

Fig. 4 shows the effect of sildenafil treatment of a horse with OA. Fig. 4A is a graph that shows clinical lameness grade of carpal joints before and after treatment with glucose/mepivacaine/sildenafil (GMS). Fig. 4B is a graph that shows decreased concentration of COMP neo-epitope in synovial fluid after treatment with glucose/mepivacaine /sildenafil (GMS). Fig. 4C is a graph that shows decreased concentration of COMP neo-epitope in serum after treatment with glucose/ mepivacaine /sildenafil (GMS) and during rehabilitation.

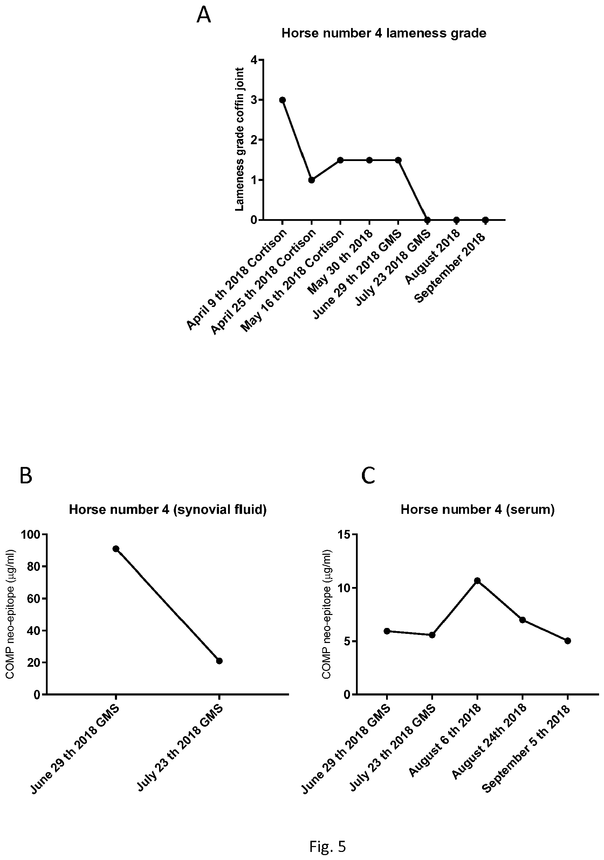

Fig. 5 shows the effect of sildenafil treatment of a horse with OA. Fig. 5A is graph that shows clinical lameness grade before and after treatment with corticosteroids or glucose/mepivacaine/sildenafil (GMS). Fig. 5B is graph that shows decreased concentration of COMP neo-epitope in synovial fluid after treatment with glucose/ mepivacaine /sildenafil (GMS). Fig. 5C is a graph that shows concentration of COMP neo-epitope in serum before and after treatment with glucose/mepivacaine/sildenafil (GMS) and during rehabilitation.

### Detailed description

There is provided a treatment of osteoarthritis, in particular a treatment by reducing the inflammation in the joint, in particular reducing inflammation signalling, such as calcium signalling, within and between chondrocytes in the joint. The treatment preferably has at least one effect of: reducing pain, inducing healing of the joint, re-growth of matrix, and decreased inflammation.

Diagnosis of OA may be done with methods known in the art, for example clinical examination, radiography as is known in the art or the use of biomarkers, such as, for example, the COMP protein fragment described in WO2017216289. The presence of the COMP fragment in a bodily fluid, for example serum or synovial fluid, indicates OA, in particular cartilage degradation during OA. The COMP fragment may be detected by using an antibody that specifically binds to a peptide comprising the amino acid sequence N-terminal-SGPTH. It is referred to WO2017216289 for details. Thus, in one embodiment, the patient is treated as described herein and the presence of a fragment of COMP is detected. The detection of the COMP fragment may be used to follow how the patient responds to the treatment.

The patient may be a human or an animal. When the patient is an animal it may preferably be a horse, but many different kinds of animals may benefit from treatment, including dogs, cats and cattle.

There is provided the use of sildenafil for the treatment of osteoarthritis.

PDE5 is an enzyme that catalyses the break-down on cGMP, which is an important second messenger in the cell. Sildenafil may compete with cGMP for binding to the active site of PDE5.

### Routes of administration

Sildenafil may be administered in any suitable manner, including orally, topically, intravenously, intradermally, or intraarticularly, intramuscular, nasal, or into the cerebrospinal fluid.

In a preferred embodiment sildenafil is delivered locally to the affected joint. In an even more preferred embodiment, the formulation is delivered intraarticularly, in particular with one or more intraarticular injections into the affected joint, i.e. injection into the joint.

Sildenafil may be administered by topical administration by, for example, transdermal administration. The transdermal formulation is specifically advantageous in regard of simplicity and from a patient comfort standpoint. It may, for instance, take the form of a transdermal patch.

In one embodiment sildenafil is delivered so that at least some systemic distribution of sildenafil is achieved. This may be achieved local administration where the drug spreads through the body or with systemic administration, for example oral administration or intravenous administration.

In addition to the methods of administration mentioned above also parenteral, intranasal, and rectal administration may be useful, as well as administration by inhalation.

The timing of the administration of sildenafil according to the invention will depend on the formulation and/or route of administration used.

When sildenafil is administered with local injection, one or more injections may be carried out. A suitable number of injections may be one, two, three, or more. The plurality of injections may be carried out with an interval of 2- 60 days, more preferably 5-30 days, even more preferably 8-25 days and most preferably 10-18 days. The recovery of the joint may be monitored using the measurement of the COMP fragment as described in WO2017216289, for example by monitoring the presence of the COMP fragment in serum or in synovial fluid. Other methods known in the art may be used, for example radiology.

In general, a pharmacologically effective amount of sildenafil is provided to a patient in need thereof. The appropriate dose range for the compound can be established in routine experiments. The dose may be lower than the sildenafil dose used to treat erectile dysfunction (which is often about 100 mg). The dose per administration of sildenafil given to a horse may be from 0.001 mg to 1000 mg, in particular 0.01 mg to 10 mg, in particular 0.01 mg to 1 mg, in particular 0.01 mg to 0.1 mg and most preferably 0.01 mg to 0.05 mg or 0.01 mg to 0.03 mg. Accordingly, the dose may be at least 0.001 mg, or at least 0.01 mg, or at least 0.1 mg, or at least 1 mg, or at least 10 mg. The dose given to human is approximately the same, in particular in the case of local administration, for example when intra-articular injection is used. Alternatively, a lower dose, which is approximately a fifth of the dose given to the horse, is given to the human.

The dose may be administered once but may be administered a suitable number of times, such as once, two times, three times, or more. The dose may be administered at least once or more preferably at least twice. In a preferred embodiment the dose is administered once, in an even more preferred embodiment two times.

Sildenafil may be combined with one or more other agents for treatment of osteoarthritis. Preferably sildenafil is combined with a local anaesthetic or glucose or both. The local anaesthetic may be a compound that binds to the intracellular portion of voltage-gated sodium channels and blocks sodium influx into nerve cells. Examples include bupivacaine, mepivacaine, lidocaine, prilocaine, ropivacaine, chloroprocaine and articaine. The local anaesthetic may be a local anaesthetic of the amino amide type or amino ester type. In a preferred embodiment both a local anaesthetic and glucose is included in the formulation.

The dose of the local anaesthetic may be a low dose. The dose may be so low that the normal inhibition of the voltage gated channel associated with the local anaesthetic is not achieved. When mepivacaine is used, the dose administered at one time may be from 1 mg to 1000 mg, in particular from 5 mg to 200 mg, even more preferred from 8 mg to 100 mg and most preferred from 10 mg to 30 mg. The dose of glucose administered at one time may be from 10 mg to 10 000 mg, in particular from 100 mg to 1000 mg, where 150 mg to 400 mg is an even more preferred range.

In one embodiment, sildenafil is administered by intraarticular injection in a dose which preferably is from 0.01 mg to 0.1 mg together with a local anaesthetic, preferably mepivacaine, preferably in a dose of from 10 mg to 200 mg, together with glucose, preferably in a dose of from 100 mg to 1000 mg. The dose may be administered once but may be administered a plurality of times, for example 2, 3, 4 or 5 times or more, where 2 or 3 times are preferred, and 2 times is most preferred. In a preferred embodiment injection is carried out 1, 2 or 3 times. The dose may be administered at least once or more preferably at least twice.

When multiple doses are given a suitable dose interval is 2-60 days, more preferably 5-30 days, even more preferably 8-25 days and most preferably from 10 to 18 days.

Recovery from OA after and during treatment may be monitored by measuring the presence of the COMP fragment, or other means, as described above.

Intraarticular injection is done as is known in the art. Injection may be done with any suitable technology, for example a disposable syringe and needle, an autoinjector, an injection pen or an infusion pump. Examples of needles useful for horse intraarticular injections are 0.90x40 mm or 70 mm (20G×1½"), 0.80x40mm, (21G × 1½") and spinal needle 1,20 mm × 152/200mm (18Gx6.00") for injection into very large joints.

An appropriate volume of injection solution is injected, where the volume is typically larger for a horse than for a human.

### Pharmaceutical composition

Sildenafil may be comprised in a suitable pharmaceutical composition. In general, the composition should be adapted to the manner of administration and various compositions comprising sildenafil are known in the art. The composition suitably contains a therapeutically effective amount of sildenafil with a suitable amount of a carrier or vehicle, in order to provide a form appropriate for the administration to the patient. The composition may have a solid, semisolid, gelatinous or hydrogel-like, a liquid and may be in the form of a tablet, a liquid, an injection solution, a suspension, a powder, a capsule, a sustained release form, a dermal patch, a gel, a cream, an ointment, a spray, or an aerosol.

A composition may, for example, comprise one or more pharmacologically acceptable excipients, including carriers, diluents, stabilizers, fillers, disintegrants, preservatives, gelling or thickening agents, release-control agents as is known in the art. Examples of compounds that may be used in the composition include: starch, cellulose and cellulose derivatives, gelatine, silica gel, magnesium carbonate, magnesium stearate, sodium stearate, glycerol monostearate, talc, sodium chloride, glycerol, glycol, water, and ethanol.

Peroral formulations of sildenafil are known in the art, for example the formulation of Viagra^{®}. For preparing further preparations for peroral administration reference is made to Pharmaceutical Dosage Forms: Tablets. Vol. 1-3, H A Lieberman et al., Eds. Marcel Dekker, New York and Basel, 1989-1990. In particular specific reference is made to chapter 7 (Special Tablets, by J W Conine and M J Pikal), chapter 8 (Chewable Tablets, by R W Mendes, OA Anaebonam and J B Daruwala), and chapter 9 (Medicated Lozenges; by D Peters). Sildenafil may be provided as a pharmaceutically acceptable salt. Sildenafil may for example be provided as sildenafil citrate.

Injection solutions comprising sildenafil are also known in the art. One example of an injection solution comprising sildenafil is Revatio^{®}. The injection solution is preferable an aqueous solution. The injection solution may comprise one or more of the following pharmacologically acceptable agents: a PH-adjusting agent, saline, osmolarity-controlling agents, stabilizers, preservatives, chelating agents, solubilizing agents, antibacterial agents and emulsifying agents. The injection solution may, for example, contain sildenafil, and pharmacologically grade water, and, optionally, a preservative. The injection solution may also comprise one or both of a local anaesthetic and glucose.

The injection solution is preferably sterile and isotonic. The injectable solution may be provided in a container such as a vial, such as an injection vial, a sachet or a cartridge. A solution for injection may be provided in an injection device for single use such as an auto injection device or an injection pen. The solution for injection may be premixed or in a form to be reconstituted, for example by addition of pharmacologically acceptable water.

When the formulation is a formulation for local delivery, for example by injection, it may also preferably comprise one of glucose and a local anaesthetic.

In one embodiment mentioned above, the formulation comprises only glucose and a local anaesthetic. When a formulation only comprises a local anaesthetic and glucose as active agents, the formulation is preferably an injection solution.

The preferred concentration of sildenafil in the injection solution is from 0.001 mg/ml to 0.1 mg/ml, more preferably 0.001 - to 0.01 mg/ml, in particular where a low dose of sildenafil is administered. The preferred concentration of local anaesthetic, for example mepivacaine, is from 0.1 mg/ml to 50mg/ml, more preferably from 1 mg/ml to 5 mg/ml. The preferred concentration of glucose is from 1 mg/ml to 200 mg/ml, more preferably from 10 mg/ml to 100 mg/ml. Where higher doses are administered, concentrations of these substances may be higher. The concentration ratio of sildenafil to mepivacaine to glucose in the formulation is preferably 0.001:1:10.

Formulations comprising sildenafil are described in WO 02/060449 and WO 02/062343. Synthesis of sildenafil is described in EP463756 and US5250534. Synthesis of Avanafil is described in US patent 7,501,409.

### Example 1 - decrease of inflammation in isolated chondrocytes

In vitro experiments suggested that treatment of horse chondrocytes with low concentration of local anaesthetic bupivacaine decreased intracellular calcium signalling and upregulated growth factors of importance for generation of cartilage. The addition of sildenafil in low concentration increased the effect.

Horse chondrocytes from OA horses were stimulated, in a fluorescence-based assay for detecting changes in intracellular Ca²⁺ over time, with ATP (10⁻⁴ M). The cells were cultivated in 5.5 mM glucose the whole cultivation period. Ca²⁺ responses were measured after the cells were incubated for 24 h with metformin (1 mM), and bupivacaine (10⁻¹² M). Controls are untreated chondrocytes. The area under the Ca²⁺ peak (AUC)(Fig 1 A) was calculated for each Ca²⁺ transient as well as the amplitude (peak) (Fig. 1 B) was expressed as the maximum increase. The cells were obtained from 4 experiments with quadruple wells in each. The level of significance was analyzed using a one-way ANOVA followed by Dunnett's multiple comparisons test. *** P*< 0.01, *** P*< 0.001. Similar data was obtained for human chondrocytes.

### Materials and Methods

### Cell model system - Chondrocyte isolation and culture

Articular cartilage samples were obtained, within 24 h of euthanasia, from the middle carpal joints of six horses (1- 8-year-old). Three horses had a normal macroscopically joint surface and three horses had a mild structural OA in the radial facet of the middle carpal bone. The horses were euthanized because of reasons unrelated to this study, and the Ethical Committee on Animal Experiments, Stockholm, Sweden, approved the study protocol (Dnr: N378/12).

Following aseptic preparation, cartilage of the dorsal aspect of the radial facet of the third carpal bone was incised with a scalpel, and full-thickness cartilage samples were collected with forceps. The tissue was placed in a sterile saline (0.9% NaCl) solution with gentamicin sulfate (50 mg/l) and amphotericin B (250 µg/ml). Cartilage samples were transported chilled (approx. 5°C) to the laboratory. Isolation and expansion of chondrocytes were performed as previously described (Ley C, Svala E, Nilton A, Lindahl A, Eloranta M, Ekman S, Skiöldebrand E.Connect Tissue Res. (2011); 52 (4): 290-300). Cells were expanded in passage 1 and cells were seeded at 20,000 cells/cm2 in chondrogenic media to maintain the phenotype, DMEM-high glucose (DMEM-HG) (Thermo Fisher Scientific; Waltham, MA, USA) supplemented with 14 µg/ml ascorbic acid (Sigma-Aldrich, St. Louis, MO, USA), 10⁻⁷ M dexamethasone (Sigma-Aldrich), 1 mg/ml human serum albumin (Equitech Bio, Kerville, TX, USA), 1 × insulin-transferrin-selenium (Gibco, Life Technologies, Carlsbad, CA, USA), 5 µg/ml linoleic acid (Sigma-Aldrich), 1 × penicillin-streptomycin (PEST) (Sigma-Aldrich), and 10 ng/ml human transforming growth factor (TGF) β-1 (R&D Systems, Abingdon, UK) for 5 days until confluence, in 96-well culture plates for Ca²⁺or in 6-well culture plates for Western blot analysis.

For restoration the cells were incubated with metformin and bupivacaine at day 4 of cultivation for 24 h. At day 5 the Ca²⁺ measurements were performed, medium collected for Western blot analysis and cells were harvested and immediately frozen at -80°C before analysis.

For anti-inflammatory restoration the cells were incubated with different combinations of pharmacological substances for 24 h.

### Drug restoration

Restoration proceeds so that the cells are incubated with the drug substances 1 mM metformin (Sigma Aldrich, St. Louis, MO, USA) for 24 h. Drug substances are: bupivacaine (Marcain) (Astra Zeneca, Södertälje, Sweden) (10⁻¹² M) (Block L, Jörneberg P, Björklund U, Westerlund A, Biber B, Hansson E. Eur. J. Neurosci. (2013); 38: 3669-3678), sildenafil citrate salt (Sigma Aldrich) (1 µM) (Nunes AKS, Raposo C, Björklund U, Cruz-Höfling MA, Peixoto CA, Hansson E, Neurosci. Lett. (2016); 630: 59-65) and 1α,25-dihydroxyvitamin D3 (calcitriol) (Sigma-Aldrich) (100 nM) (Li F, Zhang A, Shi Y, Ma Y, Du Y. Int. J. Mol. Med. (2015); 36: 967-974). The cell cultures were incubated with the above substances for 24 h before the respective experiments started.

### Calcium imaging

With a high through-put screening system for intra- and extracellular Ca²⁺-signaling, Flexstation 3 Microplate Reader (Molecular Devices, San José, USA) the cells were incubated with the Ca²⁺ sensitive probe FLIPR Calcium 6 (Molecular Devices) and exposed with different neurotransmitters; 5-HT (10⁻⁵ M), or ATP (10⁻⁴ M) all from Sigma Aldrich (Saint Louis, USA).

### Harvest of monolayer chondrocytes for RNA analysis

Cells (human chondrocytes) aimed for RNA isolation were washed with sterile PBS twice before addition of 1M DL-Dithiothreitol solution (Sigma Aldrich) in lysis buffer (Qiagen). The cells were visually inspected to ensure lysis before transfer to RNAse free, endtoxin-free sample tubes, snap frozen in liquid nitrogen, and stored at - 80°C for RNA isolation. Total RNA was extracted and purified with a commercially available kit (RNeasy mini kit, Qiagen) in accordance with the manufacturer's protocol. In brief, cells in lysis buffer were thawed and vortexed for 1 minute before 70% ethanol was added (1:1). The samples were mixed before addition to a spin column, and centrifuged. The column was then washed before genomic DNA was removed with DNase (Qiagen). The washing step was repeated with a stringent wash buffer and thereafter a mild buffer for washing membrane bound RNA. Total RNA was eluted with 50 µl RNase free water. RNA concentration was measured with a micro-volume spectrophotometer (ND-1000 spectrophotometer, NanoDrop Technologies, Wilmington, DE) and RNA quality was evaluated using the Agilent 2100 Bioanalyzer system (Agilent Technologies Inc, Palo Alto, CA).

### Quantitative Real-Time Polymerase Chain Reaction Analysis

cDNA was prepared from total RNA using High Capacity cDNA Reverse Transcription Kit (Applied Biosystems, Foster City, CA, USA). Commercially available TaqMan Gene Expression Assays all from Applied Biosystems were used (Fibroblast growth factor 18 (FGF-18) and Growth and differentiation factor-5 (GDF-5)). Samples were analyzed in duplicates. The relative comparative CT method was used to analyze the real-time polymerase chain reaction (PCR) data.

The total areas under the curve (AUC), which reflects the amount of Ca²⁺ released (Berridge MJ. Biochem. Soc. Symp. (2007); 74: 1-7), were analysed to measure the Ca²⁺ responses. The amplitude (peak) was expressed as the maximum increase.

In conclusion, the data shown in Figs. 1A-B show that bupivacaine in low concentration together with metformin reduced intracellular Ca²⁺ release in inflammatory osteoarthritic chondrocytes to a physiological level. Sildenafil increased the effect. D3 by itself did not have an effect (Fig 1E).

Figs. 1C-D show how treatment of bupivacaine and sildenafil upregulated growth factors GDF5 and FGF18 that are of importance for generation of in human chondrocytes.

### Example 2

An injection solution was prepared as follows:
1 ml mepivacaine, 20 mg/ml =20 mg
4 ml glucose 50 mg/ml = 200 mg
5 ml sterile H2O

Final concentration of mepivacaine was 2 mg/ml. Final concentration of glucose was 20 mg/ml.

10 ml was used as the dose administered to horses 1-3 by intraarticular injection in examples 4-6, below (indicated with GM (reference composition) in Figs. 2-4).

### Example 3

An injection solution was prepared as follows:
Revatio^{®}(sildenafil at 0.8 mg/ml) was diluted 200 times in sterile water to obtain 0.004 mg/ml. 5 ml of this solution (0.02 mg sildenafil) was mixed with
1 ml mepivacaine, 20 mg/ml =20 mg glucose.
4 ml glucose 50 mg/ml = 200 mg glucose.
To obtain 10 ml.

Final concentrations were: sildenafil: 0.002 mg/ml, mepivacaine: 2 mg/ml, glucose: 20 mg/ml.

10 ml (sildenafil: 0.02 mg, mepivacaine: 20 mg and glucose 200 mg) was used as the dose given to horses 2-4 in examples 4-6 by intraarticular injection (indicated with GMS (according to the invention) in Figs. 2-5).

### Example 4 - Horse number 1

Horse number 1 is a Swedish warmblood horse, mare, born 2006. It is a riding school horse that was diagnosed with bilateral lameness in both fetlock joints in January 4^{th}, 2018. The horse was treated with hyaluronic acid (HA) intra-articularly, and corticosteroids orally. The lameness was increased after second visit and the horse was then treated with a 10 ml intra-articular injection of glucose/mepivacaine (GM) of Example 1. Upon re-examination, the horse was non-lame, but was nevertheless treated once more with the same type of injection. The horse is non-lame since the first treatment with GM and has been completely non-lame at all re-examinations. The horse is back in work at the riding school in and is in full condition.

Lameness examination with flexion test and treatments have been performed by a veterinarian, and the results are shown in Fig. 2A.

Samples of synovial fluid and serum were taken from the horse at the time points indicated in Figs. 2B and C. Analysis of COMP epitope concentrations in synovial fluid and serum was carried out as in WO2017216289, and is shown in Fig. 2B and C, respectively. The presence of the COMP epitope indicates ongoing inflammation and tissue damage in the joint.

As seen in Fig. 2A, treatment with low concentration of a local anaesthetic together with glucose caused clinical recovery, associated with decrease of COMP levels to normal physiological levels as seen in Figs. 2B and 2C.

### Example 5 - Horse number 2.

Horse number 2 is a standardbred trotter, stallion, born 2012. The horse had had severe lameness since august 2016, associated with osteoarthritis in his carpal joints. He was treated with 10 ml glucose/mepivacaine (according to Example 1) intra-articular injections several times in the carpal joints since August 2016 and was considered as having failed this treatment.

He received two intra-articular 10 ml intra-articular injections with glucose/mepivacaine/sildenafil (as in Example 3) in the same joints on June 12th and 25^{th}, 2018 and was in October 2018 fully recovered with no lameness recorded at clinical examination. The stallion has been competing in trotting races in August and September 2018, and was then placed 2, 5 and 2 in the races, which indicated full recovery.

Lameness examination with flexion test and treatments have been performed by a veterinarian, and the results are shown in Fig. 3A.

Analysis of COMP epitope concentrations in synovial fluid and serum was carried out as in Example 4, and are shown in Figs. 3A and B, respectively.

As seen in Fig. 3A, addition of sildenafil to the treatment caused clinical recovery, associated with decrease of COMP levels to normal physiological levels as seen in Figs. 3B and 3C.

### Example 6 - Horse number 3

Horse number 3 is standardbred trotter, mare, born 2012. She received her first lameness in both carpal joints as a three-years old. She was treated 17 times with glucose/mepivacaine intra-articular in both carpal joints since then and was considered as having failed this treatment.

In May 2018 she received intra-articular injection with glucose/mepivacaine/sildenafil as in Example 5 in both carpal joints. The horse is now non-lame at lameness examination in May and September 2018 and is competing regularly since then.

Analysis of COMP epitope concentrations in synovial fluid and serum was carried out as in Example 4. Data from clinical lameness examination and COMP levels are shown in Figs. 4A-C. As seen in Fig. 4A, addition of sildenafil to the treatment caused clinical recovery, associated with decreased levels of COMP, as seen in Figs. 4B and 4C.

### Example 7 - Horse number 4

Horse number 4 is a pony, gelding, with severe lameness in the right coffin joint associated with osteoarthritis diagnosed with Magnetic Resonance Imagining (MRI). The horse has been treated with intra-articular injections of corticosteroids since April 2018 three times with no reduction of lameness grade. In June and July 2018, the horse was treated with glucose/mepivacaine/sildenafil (GMS) and is after that non-lame (figure 5A). COMP neo-epitope concentrations in synovial fluid and serum were clearly reduced after the first treatment with GMS (figure 5B and C). Analysis of COMP epitope concentrations in synovial fluid and serum was carried out as in Example 4.

While the invention has been described with reference to specific exemplary embodiments, the description is in general only intended to illustrate the inventive concept and should not be taken as limiting the scope of the invention. The invention is generally defined by the claims.

## Claims

1. Sildenafil for use in the treatment of osteoarthritis in humans, horse, dog or cat, where sildenafil is administered together with a local anaesthetic of the amino amide type and glucose.

2. Sildenafil for use according to claim 1 for treatment in horse.

3. Sildenafil for use according to any one of claims 1 or 2 where sildenafil is injected into the affected joint.

4. Sildenafil for use according to any one of claims 1 to 3 where a dose is administered once, twice or three times.

5. Sildenafil for use according to claim 4 where the dose is administered two times.

6. Sildenafil for use according to any one of claims 1 to 5 where the dose of sildenafil is from 0.01 mg to 0.1 mg and where the dose is administered at least once.

7. Sildenafil for use according to any one of claims 1 to 6 where the local anaesthetic is mepivacaine.

8. Sildenafil for use according to claim 7 where the dose of glucose is from 100 mg to 1000 mg and the dose of mepivacaine is from 10 mg to 200 mg.

9. Sildenafil for use according to claim 8 where the dose of glucose is from 150 mg to 400 mg and the dose of mepivacaine is from 10 mg to 30 mg.

10. A pharmaceutical composition comprising sildenafil, glucose and mepivacaine.

11. The pharmaceutical composition according to claim 10 for use in the treatment of osteoarthritis in humans, horse, dog or cat.

## Patentansprüche

1. Sildenafil zur Verwendung bei der Behandlung von Osteoarthritis bei Menschen, Pferden, Hunden oder Katzen, wobei Sildenafil zusammen mit einem Lokalanästhetikum vom Aminoamid-Typ und Glucose verabreicht wird.

2. Sildenafil zur Verwendung nach Anspruch 1 zur Behandlung bei Pferden.

3. Sildenafil zur Verwendung nach einem der Ansprüche 1 oder 2, wobei Sildenafil in das betroffene Gelenk injiziert wird.

4. Sildenafil zur Verwendung nach einem der Ansprüche 1 bis 3, wobei eine Dosis einmal, zweimal oder dreimal verabreicht wird.

5. Sildenafil zur Verwendung nach Anspruch 4, wobei die Dosis zweimal verabreicht wird.

6. Sildenafil zur Verwendung nach einem der Ansprüche 1 bis 5, wobei die Dosis Sildenafil 0,01 mg bis 0,1 mg beträgt und wobei die Dosis mindestens einmal verabreicht wird.

7. Sildenafil zur Verwendung nach einem der Ansprüche 1 bis 6, wobei das Lokalanästhetikum Mepivacain ist.

8. Sildenafil zur Verwendung nach Anspruch 7, wobei die Glucose-Dosis 100 mg bis 1000 mg beträgt und die Mepivacain-Dosis 10 mg bis 200 mg beträgt.

9. Sildenafil zur Verwendung nach Anspruch 8, wobei die Glucose-Dosis 150 mg bis 400 mg beträgt und die Mepivacain-Dosis 10 mg bis 30 mg beträgt.

10. Pharmazeutische Zusammensetzung, die Sildenafil, Glucose und Mepivacain umfasst.

11. Pharmazeutische Zusammensetzung nach Anspruch 10 zur Verwendung bei der Behandlung von Osteoarthritis bei Menschen, Pferden, Hunden oder Katzen.

## Revendications

1. Sildénafil pour utilisation dans le traitement de l'arthrose chez l'homme, le cheval, le chien ou le chat, où le sildénafil est administré avec un anesthésique local du type aminoamide et du glucose.

2. Sildénafil pour utilisation selon la revendication 1 pour le traitement du cheval.

3. Sildénafil pour utilisation selon l'une quelconque des revendications 1 ou 2 où le sildénafil est injecté dans l'articulation affectée.

4. Sildénafil pour utilisation selon l'une quelconque des revendications 1 à 3 où une dose est administrée une, deux ou trois fois.

5. Sildénafil pour utilisation selon la revendication 4 où la dose est administrée deux fois.

6. Sildénafil pour utilisation selon l'une quelconque des revendications 1 à 5 où la dose de sildénafil
est de 0,01 mg à 0,1 mg et où la dose est administrée au moins une fois.

7. Sildénafil pour utilisation selon l'une quelconque des revendications 1 à 6 où l'anesthésique local est la mépivacaïne.

8. Sildénafil pour utilisation selon la revendication 7 où la dose de glucose est de 100 mg à 1000 mg et la dose de mépivacaïne est de 10 mg à 200 mg.

9. Sildénafil pour utilisation selon la revendication 8 où la dose de glucose est de 150 mg à 400 mg et la dose de mépivacaïne est de 10 mg à 30 mg.

10. Composition pharmaceutique comprenant du sildénafil, du glucose et de la mépivacaïne.

11. Composition pharmaceutique selon la revendication 10 pour utilisation dans le traitement de l'arthrose chez l'homme, le cheval, le chien ou le chat.
